# NEW EUROPEAN PATENT SPECIFICATION

(11) **EP 0 268 347 B2**
(45) Date of publication and mention of the opposition decision: **26.02.1997**
(45) Mention of the grant of the patent: 22.07.1992
(21) Application number: 87304390.5
(22) Date of filing: 18.05.1987
(51) Int. Cl.: A61K 6/10, C08L 83/04

(54) **Two-component dental impression materials**
Zweikomponenten-Abformmassen für zahnärztliche Zwecke
Matériaux pour empreintes dentaires à deux composants

(30) Priority: 19.11.1986 US 932246; 25.03.1987 US 27499
(43) Date of publication of application: 25.05.1988
(73) Proprietor: Kerr Manufacturing Company (a Delaware corporation), Romulus, Michigan 48174 (US)
(72) Inventor: Waller, Duncan Ewan, Ypsilanti, MI 48174 (US); Lovshe, Laurie Denise, Plymouth, MI 48170 (US)
(74) Representative: Ackroyd, Robert

(56) References cited:
- EP-A- 0 046 907
- EP-A- 0 117 056
- EP-A- 0 231 420
- WO-A-87/03001
- DE-A- 2 926 405
- US-A- 4 020 014
- H. RÖMPP: "Chemie Lexikon", 5th edition, 1962, pages 3952-3953, Franckh'sche Verlagshandlung, Stuttgart, DE;
- Handbuch der Präparativen Anorganischen Chemie, G.Brauer, F.Enke Verlag Stuttgart, Dritter Band, 3. Auflage, pp.1706-1707 (1981)
- The Journal of Prostetic Dentistry 42, pp.342-347 (1979)
- Meyers Grosses Universal Lexikon; Band 11, pp.66-67 (1984), Bibliographisches Institut Mannheim/Wien/Zürich
- H.Römpp Chemie Lexikon, 6. Auflage, pp. 4980-4982 (1966), Franck'sche Verlagsbuchhandlung Stuttgart
- Ullmanns Encyklopädie der technischen Chemie, W. Foerst Hrsg., 3. Auflage, Band 14, p. 18 (1963), Urban & Schwarzenberg - München - Berlin
- Glossary of Chemical Terms, C.A.Hampel and G.G.Hawlwy, 2nd ed., pages 1 and 8 (1993), Van Nostrand Reinhold Co.
- Hawley's Chemical Dictionary, 11th ed., page 927 (1981), Van Nostrand Reinhold, New York

## Description

The present invention relates to dental impression materials of the kind comprising two components which are mixed together in use and is concerned more particularly with two-component dental impression materials which comprise polyvinylsiloxane elastomers, the setting and hardening of which is catalysed by means of platinum black.

The room temperature vulcanized liberation of small quantities of hydrogen gas from RTV addition-cured polyvinylsiloxane elastomers, due to reaction between the platinum catalyst and hydrofunctional polydimethylsiloxane, is a recognised problem. The evolution of hydrogen gas results in the formation of pores in the model formed from the impression, producing an undesirable pitted surface.

This problem is described in US-A-4,273,902 and a solution to it is disclosed based upon the use of 0.5 ppm or more of finely-divided palladium and/or a finely-divided palladium alloy containing 10% by weight or more of palladium, without inhibiting the addition reacton. Various other elemental metals are cited in this publication, including platinum, but it is stated that these are inferior to palladium and fail to eliminate the undesirable pores in the surface of the resulting model.

The adsorption of hydrogen by palladium is variously quoted as 592, 935 and even 2952 times its own volume, (see J. W. Mellor, Inorganic Chemistry, Vol XVII, 1947, page 616 et seq.,) while the adsorption of hydrogen by platinum black is quoted as 310 volumes in Vol XVI. It therefore becomes apparent that these adsorption variabilities arise from differences in the available surface areas of the finely-divided metals, but are not significant in the case of the polyvinylsiloxane elastomer application since, if sufficiently finely-divided and present in adequate concentration, the adsorption saturation level will never be reached.

Another problem associated with polyvinylsiloxane dental impression materials is their extremely hydrophobic characteristics, which lead to comparatively large bubble-shaped artifacts in the surface of such impressions.

Accordingly, it has been discovered that when samples of extremely finely-divided platinum black are obtained with the highest possible surface area, specifically 24 m²/g, and are compared with equal weight percent concentrations of palladium black, by incorporation in identical polyvinylsiloxane elastomer pastes, they are equally and completely effective, down to a concentration level of about 0.2 ppm by weight. An effective concentration range for the platinum black is from 0.2 to 20,000 ppm by weight. The size range for the platinum black should be as small as possible. A preferred size range for the platinum black is from 18 to 28 Ångstrom Units.

The present invention thus provides a two-component dental impression material, which comprises a base paste and a catalyst paste, each containing a polyvinylsiloxane elastomer, characterised in the catalyst paste contains in addition platinum black as a hydrogen gas adsorption agent and does not contain particulate palladium and/or any particulate palladium alloy.

The use of platinum black in accordance with the present invention allows the control or prevention of outgassing in all addition-cured polyvinylsiloxane elastomers, which are primarily used in making dental impressions.

The platinum black is used to absorb gaseous hydrogen generated during the curing reaction and generally is present at a concentration level in the range from 0.2 to 20,000 ppm. A preferred concentration range is from 0.001 to 0.01 weight percent (i.e., 1,000 to 10,000 ppm). The platinum black is added or blended with the catalyst paste in any convenient manner, such as described in US-A-4,273,902.

It has also been discovered, in accordance with a preferred embodiment of the invention, that the extremely hydrophobic characteristics of polyvinylsiloxane dental impression materials, which lead to comparatively large bubble-shaped artifacts in the surface of impressions made with them, can be overcome by the incorporation of relatively small quantities of selected surfactants.

In preferred two-component dental impression materials according to this invention, both of the base and catalyst pastes contain vinyl polydimethylsiloxane and silica or other similar inert filler(s) and the base paste contains a moiety of hydrofunctional polydimethylsiloxane, whereas the catalyst contains both a chloroplatinic acid catalyst complex and platinum black, to adsorb any gaseous hydrogen formed during mixing of the pastes and curing of the impression.

A cured impression is produced by mixing the base paste and the catalyst, such as that illustrated in Example I below, in a 1:1 or other suitable ratio to form a homogeneous plastic mass, which is applied over the dentition and adjacent gingival tissue and allowed to cure during a time frame of several minutes prior to mouth removal. Models made from impressions taken with the platinum black-containing materials of the invention, immediately after mouth removal, are found to be free from pores in all cases, at or above the 0.005 weight percent platinum level.

Impressions and resulting moulds or models formed using the compositions of the present invention result in a surface smoothness which is significantly better than upon those formed without the use of platinum black.

Another problem associated with polyvinylsiloxane dental impression materials is caused by their extremely hydrophobic characteristics, which can lead to the formation of comparatively large bubble-shaped artifacts in the surface of such impressions.

US-A-4,600,751 discloses the controlled release of bioactive agents and serves to demonstrate the synthesis of silicone-based prepolymers which are extremely hydrophilic.

In dentistry, impression materials, particularly for making crown and bridge impressions, require a high degree of dimensional stability, to facilitate the production of extremely accurate prostheses, for which the techniques of US-A-4,600,751 are totally unsuitable, because high water sorption is consistent with swelling and distortion of these polymers. Furthermore, there are other definite advantages to dental impressions with low water sorption, such as ease of disinfection and sterilization.

With respect to the problem associated with these hydrophobic characteristics, in the dental impression materials of the present invention, it has been found possible to utilize several species of hydroyhilic compounds as low-concentration additives to conventional extremely hydrophobic addition-cured polyvinylsiloxane dental impression materials, so as to impart truly hydrophilic surface characteristics to these materials. This prevents the formation of comparatively large bubble-shaped artifacts in the surface of these impressions, due to the effects of surface tension at a strongly hydrophobic/hydrophilic interface causing a film of moisture to form water droplets.

In a preferred embodiment of this invention, any one or more of the following surfactant type compounds are added to conventional addition-cured polyvinylsiloxane dental impression materials in relatively low concentrations of about 1.0% to 10.0% by weight.

| COMPOUND NAME | CAS NO. WHERE KNOWN |
|---|---|
| n-dodecyl tetradecyl hexadecyl alcohol ethoxylate | 68551-12-2 |
| polyethylene glycol monolaurate | 9004-81-3 |
| polyethylene glycol dilaurate | |
| polyethylene glycol monoleate | |
| polyethylene glycol dioleate | |
| polyethylene glycol monotallate | |
| polyethylene glycol ditallate | |
| sorbitan monolaurate | |
| sorbitan trioleate | |
| sorbitan monotallate | |
| sorbitan tritallate | |
| polyethylene glycol glycerol coceate | |
| caprylic triglyceride | |
| polyoxyethylene tridecyl alcohol | 24938-91-8 |
| polyoxyethylene lauryl ether | 5274-68-0 |
| nonylphenoxypoly (ethyleneoxy) ethanol | 9016-45-9 |
| polyoxyethylene sorbitan monolaurate | |
| sorbitan monolaurate polyoxyethylene polysorbate | 9005-64-5 |
| polyoxyethylene oleyl alcohol | |

The above list of suitable surfactants is not exhaustive, but serves to illustrate twenty representative examples of the many chemical permutations possible with the family of polyol fatty acid ester and ethoxylated ester type surfactants useful for this invention.

The effectiveness of these surfactant additives is readily apparent in their ability to reduce the aqueous contact angle with cured or uncured addition-curable polyvinylsiloxane dental impression materials at temperatures in the range from 95°-110°C down to 30°C or less. Upon contact of the dental impression materials with the dentition and gingiva, any moisture present on the surface of these oral tissues is readily miscible with the surfactant moiety available at the surface of the impression material, which provides uniform wetting of the interface between the impression material and the oral tissues, thereby dramatically reducing surface tension.

The following Examples illustrate embodiments of the present invention. The concentrations are in weight percent unless otherwise stated.

### EXAMPLE 1

### Embodiments containing platinum black for gaseous adsorption

| BASE PASTE (VERY HEAVY VISCOSITY) | |
|---|---|
| | WT% |
| vinyl polydimethylsiloxane | 20% |
| hydropolydimethyl siloxane | 3% |
| silica filler | 70% |
| liquid petroleum or other inert plasticizer | 7% |

| CATALYST PASTE (VERY HEAVY VISCOSITY) | |
|---|---|
| | WT% |
| vinyl polydimethylsiloxane | 20% |
| *cyclic vinyl siloxane | 0.4% |
| **chloroplatinic acid complex | 1.0% |
| liquid petroleum or other | 7% |
| inert plasticizer | 7% |
| silica or other inert filler | 71.6% |

| | |
|---|---|
| * Available under the tradename PSW 2204 from Petrarch Silicones of Bristol, PA. | |
| ** Available under the tradename PSW 2206 from Petrarch Silicones of Bristol, PA. | |

The following illustrates suitable compositional ranges for the components of the base and catalyst pastes.

| BASE PASTE | WT% |
|---|---|
| vinyl polydimethylsiloxane | 10-60 |
| hydropolydimethyl siloxane | 1-10 |
| silica filler | 20-80 |
| liquid petroleum | 0-15 |

| CATALYST PASTE | WT% |
|---|---|
| vinyl polydimethylsiloxane | 10-60 |
| cyclic vinyl siloxane | 0-2 |
| chloroplatinic acid complex | 0.1-5.0 |
| platinum black | 0.2-20,000 ppm |
| plasticizer | 0-15 |
| filler | 20-80 |

The following illustrates suitable compositional ranges for a cured dental impression material of the present invention.

| VERY HEAVY VISCOSITY MATERIAL (PUTTY) | |
|---|---|
| | WT% |
| vinyl polydimethylsiloxane | 10-60 |
| cyclic vinyl siloxane | 0-1 |
| hydropolydimethyl siloxane | 0.5-5.0 |
| filler | 20-80 |
| chloroplatinic acid complex | 0.05-2.5 |
| platinum black | 0.1-10,000 ppm |
| plasticizer | 0-15 |
| surfactant (optional but preferred) | 2-10 |

### Embodiments using surfactants to confer hydrophilic characteristics

### EXAMPLE 2

| Low viscosity catalyst paste with surfactant | |
|---|---|
| | WT% |
| vinyl functional polydimethylsiloxane | 51.785 |
| siliceous filler | 43.0 |
| n-dodecyl tetradecyl hexadecyl alcohol ethoxylate | 4.0 |
| pigment | 1.0 |
| cyclic vinyl functional prepolymer | 0.2 |
| chloroplatinic acid catalyst complex | 0.01 |
| platinum black | 0.005 |
| | $\overline{\text{100.00}}$ |

Excellent hydrophilic surface characteristics, but unpleasant taste.

### EXAMPLE 3

| Low viscosity base paste with surfactant | |
|---|---|
| vinyl functional polydimethylsiloxane | 46.0 |
| siliceous filler | 42.0 |
| hydrofunctional polydimethylsiloxane | 7.0 |
| pigment | 2.0 |
| nonylphenoxypoly (ethyleneoxy) ethanol | 3.0 |
| | $\overline{\text{100.0}}$ |

Excellent hydrophilic surface characteristics and acceptable taste.

### EXAMPLE 4

| Low viscosity base paste with surfactant | |
|---|---|
| vinyl functional polydimethylsiloxane | 46.0 |
| siliceous filler | 41.0 |
| hydrofunctional polydimethylsiloxane | 7.0 |
| n-dodecyl tetradecyl hexadecyl alcohol ethoxylate | 4.0 |
| pigment | 2.0 |
| | $\overline{\text{100.00}}$ |

Excellent hydrophilic surface characteristics, but unpleasant taste.
Although the above examples are restricted to low viscosity polyvinylsiloxane dental impression materials, the same family of polyol fatty acid esters and ethoxylated ester-type surfactants can be used as additives in the regular, heavy and even putty viscosities of the same type of material with equally effective results.

The following illustrates suitable compositional ranges for the components of the base and catalyst pastes of Examples 2 to 4.

| BASE PASTES | WT% |
|---|---|
| vinyl polydimethylsiloxane | 25-75 |
| hydropolydimethyl siloxane | 1-12 |
| siliceous filler(s) | 25-75 |
| surfactant(s) | 1-10 |
| pigment(s) | 0.5 |

| CATALYST PASTES | WT% |
|---|---|
| vinyl polydimethylsiloxane | 25-75 |
| cyclic vinyl siloxane | 0.5 |
| siliceous filler(s) | 25-75 |
| surfactant(s) | 1-10 |
| pigment(s) | 0.5 |
| chloroplatinic acid complex | 0.1-5.0 |
| platinum black | 0.2-20,000 ppm |

The following illustrates suitable compositional ranges for cured dental impression material(s) of Examples 2 to 4 of the present invention:

| LOW VISCOSITY MATERIAL (LIGHT BODY OR WASH) | |
|---|---|
| | WT% |
| Vinylpolydimethylsiloxane | 25-75 |
| hydro polydimethylsiloxane | 0.5-5.0 |
| cyclic vinyl siloxane | 0-1 |
| siliceous filler(s) | 25-75 |
| surfactant(s) | 1-10 |
| pigment(s) | 0-5 |
| chloroplatinic acid complex | 0.05-2.5 |
| platinum black | 0.01-10,000 ppm |

The following is a preferred embodiment of the present invention which exhibits the desired properties with respect to controlling outgassing and hydrophillic characteristics:

| LOW VISCOSITY BASE PASTE WITH SURFACTANT | |
|---|---|
| | WT% |
| vinyl functional polydimethylsiloxane (4000 cps) | 46.0 |
| calcium silicate filler | 42.0 |
| hydrofunctional polydimethylsiloxane | 7.0 |
| nonylphenoxypoly (ethyleneoxy) ethanol | 3.0 |
| cobalt blue pigment | 2.0 |
| | $\overline{\text{100.00}}$ |

| LOW VISCOSITY CATALYST PASTE WITH PLATINUM | |
|---|---|
| vinyl functional polydimethylsiloxane (4000 cps) | 54.73 |
| calcium silicate filler | 43.98 |
| cobalt blue pigment | 1.0 |
| cyclic vinyl functional prepolymer | 0.27 |
| chloroplatinic acid catalyst complex | 0.01 |
| platinum black (24 m²/g) | 0.01 |
| | $\overline{\text{100.00}}$ |

The following is a typical method used in preparing the compounds of the present invention. Other methods and compounds, such as those set forth in the above-mentioned US-A-4,273,902, may also be used in conjunction with this invention.

### BASE PASTE

In a double planetary mixer, the three liquid components, viz. the vinyl functional polydimethylsiloxane, hydrofunctional polydimethylsiloxane and nonylphenoxypoly(ethyleneoxy) ethanol, are first blended together. The pigment is added and then the mixture is reblended to disperse the pigment. The filler is then added and then the whole is mixed until thoroughly homogeneous, to form a low-viscosity fluid paste. The resulting paste is then rollmilled to maximize its homogeneity, followed by packaging as desired.

### CATALYST PASTE

Preblends are made of (a) the chloroplatinic acid complex with a portion of the vinyl functional polydimethylsiloxane and (b) the platinum black with a portion of the calcium silicate filler. The three liquid components, viz. the catalyst complex preblend, the balance of the vinyl functional polydimethylsiloxane and the cyclic vinyl prepolymer, are then blended together. The pigment is then added and the mixture is reblended to disperse it. The platinum black preblend is then added, followed by the balance of the filler and mixing is continued until thoroughly homogeneous. Rollmilling to maximize homogeneity is then carried out, followed by packaging as desired.

## Claims

1. A two-component dental impression material, which comprises a base paste and a catalyst paste, each containing a polyvinylsiloxane elastomer, characterised in that the catalyst paste contains in addition platinum black as a hydrogen gas adsorption agent, and does not contain particulate palladium and/or any particulate palladium alloy.

2. A dental impression material according to claim 1, wherein the platinum black is present in an amount of up to 20,000 ppm by weight of the catalyst paste.

3. A dental impression material according to claim 1 or 2, which comprises:
| BASE PASTE | WT% |
|---|---|
| vinyl polydimethylsiloxane | 10-60 |
| hydropolydimethylsiloxane | 1-10 |
| silica filler | 20-80 |
| liquid petroleum | 0-15 |
| CATALYST PASTE | |
|---|---|
| vinyl polydimethylsiloxane | 10-60 |
| cyclic vinyl siloxane | 0-2 |
| chloroplatinic acid complex | 0.1-5.0 |
| platinum black | 0.2-20,000 ppm |
| plasticizer | 0-15 |
| filler | 20-80 |

4. A dental impression material according to claim 1, 2 or 3, wherein the platinum black has a size range of 18 to 28 Ångstroms.

5. A dental impression material according to any preceding claim, which contains a hydrophilic surfactant.

6. A dental impression material according to claim 5, where the surfactant is selected from polyol fatty acid esters and ethoxylated esters.

7. A dental impression material according to claim 6, wherein the surfactant comprises at least one compound selected from:
n-dodecyl tetradecyl hexadecyl alcohol ethoxylate,
polyethylene glycol monolaurate,
polyethylene glycol dilaurate,
polyethylene monoleate,
polyethylene glycol dioleate,
polyethylene glycol monotallate,
polyethylene glycol ditallate,
sorbitan monolaurate,
sorbitan monoleate,
sorbitan trioleate,
sorbitan monotallate,
sorbitan tritallate,
polyethylene glycol glycerol cocoeate,
caprylic trigylceride,
polyoxyethylene tridecyl alcohol,
polyoxyethylene lauryl ether,
nonylphenoxypoly-(ethyleneoxy)-ethanol,
polyoxyethylene sorbitan monolaurate,
sorbitan monolaurate polyoxyethylene polysorbate
and
polyoxyethylene oleyl alcohol.

8. A dental impression material according to any of claims 5 to 7, which comprises:
| BASE PASTE | WT% |
|---|---|
| vinyl polydimethylsiloxane | 10-60 |
| hydropolydimethyl siloxane | 1-10 |
| silica filler | 20-80 |
| liquid petroleum | 0-15 |
| surfactant | 1-10 |
| CATALYST PASTE | |
|---|---|
| vinyl polydimethylsiloxane | 10-60 |
| cyclic vinyl siloxane | 0-2 |
| chloroplatinic acid complex | 0.1-5.0 |
| platinum black | 0.2-20,000 ppm |
| plasticizer | 0-15% |
| filler | 20-80 |

9. A cured dental impression, which comprises:
| | WT% |
|---|---|
| vinyl polydimethylsiloxane | 10-60 |
| cyclic vinyl siloxane | 0-1 |
| hydropolydimethyl siloxane | 0.5-5.0 |
| filler | 20-80 |
| chloroplatinic acid complex | 0.05-2.5 |
| platinum black | 0.1-10,000 ppm |
| plasticizer | 0-15 |
and does not contain particulate palladium and/or any particulate palladium alloy.

10. A dental impression according to claim 9, which also contains 1 to 10 by weight of at least one hydrophilic surfactant.

11. A polyvinylsiloxane catalyst paste, which comprises a polyvinylsiloxane elastomer, characterised in that up to 20,000 ppm of finely-divided platinum black is included, which functions to adsorb hydrogen gas generated on reaction of the catalyst paste with a hydrofunctional polydimethylsiloxane, and in that the paste does not contain particulate palladium and/or any particulate palladium alloy.

12. A catalyst paste according to claim 11, wherein the platinum black is present in a concentration in the range from 0.2 to 20,000 ppm by weight.

13. A catalyst paste according to claim 11 or 12, wherein the platinum black has a size range of 18 to 28 Ångstroms.

14. A catalyst paste according to any of claims 11 to 13, which has the following composition:
vinyl polydimethylsiloxane
cyclic vinyl siloxane
chloroplatinic acid complex
platinum black
plasticizer
filler.

15. A catalyst paste according to any of claims 11 to 14, which contains a hydrophilic surfactant selected from polyol fatty acid esters and ethoxylated esters.

16. A catalyst paste according to claim 15, wherein the surfactant comrpises at least one compound selected from:
n-dodecyl tetradecyl hexadecyl alcohol ethoxylate,
polyethylene glycol monolaurate,
polyethylene glycol dilaurate,
polyethylene glycol monoleate,
polyethylene glycol dioleate,
polyethylene glycol monotallate,
polyethylene glycol ditallate,
sorbitan monolaurate,
sorbitan monoleate,
sorbitan trioleate,
sorbitan monotallate,
sorbitan tritallate,
polyethylene glycol glycerol coceate,
caprylic trigylceride,
polyoxyethylene tridecyl alcohol,
polyoxyethylene lauryl ether,
nonylphenoxypoly (ethyleneoxy) ethanol,
polyoxyethylene sorbitan monolaurate,
sorbitan monolaurate polyoxyethylene polysorbate
and
polyoxyethylene oleyl alcohol.

## Patentansprüche

1. Zweikomponenten-Abdruckmasse für zahnärztliche Zwecke, die eine Basispaste und eine Katalysatorpaste aufweist, von denen jede ein Polyvinylsiloxan-Elastomer enthält, **dadurch gekennzeichnet,** daß die Katalysatorpaste zusätzlich Platinmohr als Adsorptionsmittel für Wasserstoffgas enthält und nicht Palladiumpartikel und/oder irgendeine Palladiumpartikellegierung enthält.

2. Abdruckmasse nach Anspruch 1, wobei das Platinmohr in einer Menge bis zu 20,000 ppm Gewichtsteilen der Katalysatorpaste vorhanden ist.

3. Abdruckmasse nach Anspruch 1 oder 2, welche enthält:
| BASISPASTE | Gewichtsprozent |
|---|---|
| Vinylpolydimethylsiloxan | 10-16 |
| Hydropolydimethylsiloxan | 1-10 |
| Quarz-Füllstoff | 20-80 |
| Flüssiges Petroleum | 0-15 |
| KATALYSATORPASTE | |
|---|---|
| Vinylpolydimethylsiloxan | 10-60 |
| Zyklisches Vinylsiloxan | 0-2 |
| Chloroplatinsäure-Komplex | 0.1-5.0 |
| Platinmohr | 0.2-20,000 ppm |
| Weichmacher | 0-15 |
| Füllstoff | 20-80 |

4. Abdruckmasse nach Anspruch 1,2 oder 3, wobei das Platinmohr einen Größenbereich von 18 bis 28 · 10⁻¹⁰ m aufweist.

5. Abdruckmasse nach einem der vorhergehenden Ansprüche, welches einen hydrophilen oberflächenaktiven Stoff enthält.

6. Abdruckmasse nach Anspruch 5, wobei der oberflächenaktive Stoff aus Polycl-Fettsäureestern und ethoxilierten Estern ausgewählt ist.

7. Abdruckmasse nach Anspruch 6, wobei der oberflächenaktive Stoff mindestens eine der folgenden Verbindungen enthält:
n-Dodecyl-Tetradecyl-Hexadecyl-Alkohol-Äthoxylat,
Polyäthylen-Glycol-Monolaurat,
Polyäthylen-Glycol-Dilaurat,
Polyäthylen-Monoleat,
Polyäthylen-Glycol-Dioleat,
Polyäthylen-Glycol-Monotallat,
Polyäthylen-Glycol-Ditallat,
Sorbitan-Monolaurat,
Sorbitan-Monoleat,
Sorbitan-Trioleat,
Sorbitan-Monotallat,
Sorbitan-Tritallat,
Polyäthylen-Glycol-Glycerol-Cocoeat,
Capryl-Triglycerid,
Polyoxyäthylen-Tridecyl-Alkohol,
Polyoxyäthylen-Lauryl-Äther,
Nonylphenoxypoly-(Äthylenoxy)-Äthanol,
Polyoxyäthylen-Sorbitan-Monolaurat,
Sorbitan-Monolaurat-Polyoxyäthylen-Polysorbat
und
Polyoxyäthylen-Oleyl-Alkohol.

8. Abdruckmasse nach Anspruch der Ansprüche 5 bis 7, mit:
| BASISPASTE | Gewichtsprozent |
|---|---|
| Vinyl-Polydimethylsiloxan | 10-60 |
| Hydropolydimethylsiloxan | 1-10 |
| Quarz-Füllstoff | 20-80 |
| Flüssiges Petroleum | 0-15 |
| Oberflächenaktiver Stoff | 1-10 |
| KATALYSATORPASTE | |
|---|---|
| Vinylpolydimethylsiloxan | 10-60 |
| Zyklisches Vinylsiloxan | 0-2 |
| Chloroplatinsäure-Komplex | 0.1-5.0 |
| Platinmohr | 0.2-20,000 ppm |
| Weichmacher | 0-15 |
| Füllstoff | 20-80 |

9. Gehärtete Abdruckmasse für zahnärztliche Zwecke, mit:
| | Gewichtsprozent |
|---|---|
| Vinylpolydimethylsiloxan | 10-60 |
| Zyklisches Vinylsiloxan | 0-1 |
| Hydropolydimethylsiloxan | 0.5-5.0 |
| Füllstoff | 20-80 |
| Chloroplatinsäure-Komplex | 0.05-2.5 |
| Platinmohr | 0.1-10,000 ppm |
| Weichmacher | 0-15 |
und nicht Palladiumpartikel und/oder irgendeine Palladiumpartikellegierung enthält.

10. Abdruckmasse nach Anspruch 9, die auch 1 bis 10 Gewichtsprozent mindestens eines hydrophilen oberflächenaktiven Stoffs enthält.

11. Polyvinylsiloxan-Katalysatorpaste, die ein Polyvinylsiloxan-Elastomer enthält,
dadurch gekennzeichent, daß bis zu 20,000 ppm von fein verteiltem Platinmohr enthalten ist, um Wasserstoffgas zu absorbieren, das bei einer Reaktion der Katalysatorplaste mit einem hydrofunktionellen Polydimethylsiloxan erzeugt wird und daß die Paste nicht Palladiumpartikel und/oder irgendeine Palladiumpartikellegierung enthält.

12. Katalysatorpaste nach Anspruch 11, wobei das Platinmohr in dem Bereich von 0.2 bis 20,000 ppm enthalten ist.

13. Katalysatorpaste nach Anspruch 11 oder 12, wobei das Platinmohr einen Größenbereich von 18 bis 28 · 10⁻¹⁰ m aufweist.

14. Katalysatorpaste nach einem der Ansprüche 11 bis 13, mit der Zusammensetzung:
Vinylpolydimethylsiloxan
Zyklisches Vinylsiloxan
Chloroplatinsäure-Komplex
Platinmohr
Weichmacher
Füllstoff.

15. Katalysatorpaste nach einem der Ansprüche 11 bis 14, die einen hydrophilen oberflächenaktiven Stoff enthält, der ausgewählt ist aus Polyolfettsäureestern und äthoxylierten Estern.

16. Katalysatorpaste nach Anspruch 15, wobei der oberflächenaktive Stoff mindestens eine der folgenden Verbindungen enthält:
n-Dodecyl-Tretadecyl-Hexadecyl-Alkohol-Äthoxylat,
Polyäthylen-Glytol-Monolaurat,
Polyäthylen-Glycol-Dilaurat,
Polyäthylen-Glycol-Monoleat,
Polyäthylen-Glycol-Dioleat,
Polyäthylen-Glycol-Monotallat,
Polyäthylen-Glycol-Ditallat,
Sorbitan-Monolaurat,
Sorbitan-Monoleat,
Sorbitan-Trioleat,
Sorbitan-Monotallat,
Sorbitan-Tritallat,
Polyäthylen-Glycol-Glycerol-Coceat,
Capryl-Triglycerid,
Polyoxyäthylen-Tridecyl-Alkohol,
Polyoxyäthylen-Lauryl-Äther,
Nonylphenoxypoly(Äthylenoxy)-Äthanol,
Polyoxyäthylen-Sorbitan-Monolaurat,
Sorbitan-Monolaurat-Polyoxyäthylen-Polysorbat
und
Polyoxyäthylen-Oleyl-Alkohol.

## Revendications

1. Matière à deux composants pour empreinte dentaire, cette matière comprenant une pâte de base et une pâte de catalyseur, chaque pâte comprenant un polyvinylsiloxane élastomère, matière caractérisée en ce que la pâte de catalyseur contient en outre du noir de platine, comme agent d'adsorption de l'hydrogène gazeux, et ne contient pas de palladium particulaire et/ou un alliage quelconque de palladium particulaire.

2. Matière pour empreinte dentaire selon la revendication 1, dans laquelle le noir de platine est présent en une quantité allant jusqu'à 20 000 parties par million en poids de la pâte du catalyseur.

3. Matière pour empreinte dentaire selon la revendication 1 ou 2, qui comprend :
| Pâte de base | % en poids |
|---|---|
| Poly(vinyl)diméthylsiloxane | 10-60 |
| Hydropolydiméthylsiloxane | 1-10 |
| Silice de charge | 20-80 |
| Pétrole liquide | O-15 |
| Pâte de catalyseur | |
|---|---|
| Poly(vinyl)diméthylsiloxane | 10-60 |
| Vinylsiloxane cyclique | 0-2 |
| Complexe d'acide chloroplatinique | 0,1-5,0 |
| Noir de platine | 0,2-20 000 ppm |
| Plastifiant | 0-15 |
| Charge | 20-80 |
| | |
|---|---|
| (ppm : parties par million) | |

4. Matière pour empreinte dentaire selon la revendication 1, 2 ou 3, dans laquelle la taille du noir de platine se situe entre 18 et 28 Ångstroms (180 à 280 nm).

5. Matière pour empreinte dentaire selon l'une quelconque des revendications précédentes, qui contient un tensio-actif hydrophile.

6. Matière pour empreinte dentaire selon la revendication 5, dans laquelle le tensio-actif est choisi parmi des esters de polyols et d'acides gras et des esters éthoxylés.

7. Matière pour empreinte dentaire selon la revendication 6, dans laquelle le tensio-actif comprend au moins un composé choisi parmi :
un éthoxylate d'alcool n-dodécylique, tétradécylique, hexadécylique ; du monolaurate de polyéthylène-glycol,
du dilaurate de polyéthylène-glycol,
du monooléate de polyéthylène-glycol
du dioléate de polyéthylène-glycol,
du monotallate de polyéthylène-glycol,
du ditallate de polyéthylène-glycol,
du monolaurate de sorbitanne,
du monooléate de sorbitanne,
du trioléate de sorbitanne,
du monotallate de sorbitanne,
du tritallate de sorbitanne,
du "cocoate" de polyéthylène-glycol-glycérol,
du triglycéride caprylique,
de l'alcool polyoxyéthylène tridécylique,
de l'éther laurylique de polyoxyéthylène,
du nonylphénoxypoly-(éthylène-oxy)-éthanol,
du monolaurate de polyoxyéthylène-sorbitanne,
du polyoxyéthylène polysorbate monolaurate de sorbitanne, et
de l'alcool polyoxyéthylène-oléylique.

8. Matière pour empreinte dentaire selon l'une quelconque des revendications 5 à 7, qui comprend :
| Pâte de base | % en poids |
|---|---|
| Poly(vinyl)diméthylsiloxane | 10-60 |
| Hydropolydiméthylsiloxane | 1-10 |
| Silice de charge | 20-80 |
| Pétrole liquide | 0-15 |
| Tensio-actif | 1-10 |
| Pâte de catalyseur | |
|---|---|
| Poly(vinyl)diméthylsiloxane | 10-60 |
| Vinylsiloxane cyclique | 0-2 |
| Complexe d'acide chloroplatinique | 0,1-5,0 |
| Noir de platine | 0,2-20 000 ppm |
| Plastifiant | 0-15 |
| Charge | 20-80 |

9. Empreinte dentaire durcie, qui comprend :
| | % en poids |
|---|---|
| Poly(vinyl)diméthylsiloxane | 10-60 |
| Vinylsiloxane cyclique | 0-1 |
| Hydropolydiméthylsiloxane | 0,5-5,0 |
| Charge | 20-80 |
| Complexe d'acide chloroplatinique | 0,05-2,5 |
| Noir de platine | 0,1-10 000 ppm |
| Plastifiant | 0-15 |
et qui ne contient pas de palladium particulaire et/ou un alliage quelconque de palladium particulaire.

10. Empreinte dentaire selon la revendication 9, qui contient également 1 à 10 % en poids d'au moins un tensio-actif hydrophile.

11. Pâte de catalyseur comportant du polyvinylsiloxane, qui comprend un polyvinylsiloxane élastomère et est caractérisée en ce qu'elle comporte jusqu'à 20 000 parties par million (ppm) de noir de platine finement divisé, ayant pour rôle d'adsorber l'hydrogène gazeux engendré lors de la réaction de la pâte de catalyseur avec un polydiméthylsiloxane contenant des groupes hydro(xyles) fonctionnels, et en ce que la pâte ne contient pas de palladium particulaire et/ou un alliage quelconque de palladium particulaire.

12. Pâte de catalyseur selon la revendication 11, dans laquelle le noir de platine est présent en une concentration comprise entre 0,2 et 20 000 ppm en poids.

13. Pâte de catalyseur selon la revendication 11 ou 12, dans laquelle la taille du noir de platine se situe entre 18 et 28 Ångstroms (180 à 280 nm).

14. Pâte de catalyseur selon l'une quelconque des revendications 1 à 13, qui a la composition suivante :
poly(vinyl)diméthylsiloxane,
vinylsiloxane cyclique,
complexe d'acide chloroplatinique,
noir de platine,
plastifiant,
charge
et ne contient pas de palladium particulaire et/ou un alliage quelconque de palladium particulaire.

15. Pâte de catalyseur selon l'une quelconque des revendications 11 à 14, qui contient un tensio-actif hydrophile choisi parmi les esters de polyols et d'acides gras et des esters éthoxylés.

16. Pâte de catalyseur selon la revendication 15, dans laquelle le tensio-actif compte au moins un compose choisi parmi :
un éthoxylate d'alcool n-dodécylique, tétradécylique, hexadécylique,
du monolaurate de polyéthylène-glycol,
du dilaurate de polyéthylène-glycol,
du monooléate de polyéthylène-glycol,
du dioléate de polyéthylène-glycol,
du monotallate de polyéthylène-glycol,
du ditallate de polyéthylène-glycol,
du monolaurate de sorbitanne,
du monooléate de sorbitanne,
du trioléate de sorbitanne,
du monotallate de sorbitanne,
du tritallate de sorbitanne,
du "cocoate" de polyéthylène-glycol-glycérol,
du triglycéride caprylique,
de l'alcool polyoxyéthylène tridécylique,
de l'éther polyoxyéthylénique de lauryle,
du nonylphénoxypoly(éthylène-oxy)-éthanol,
du monolaurate de polyoxyéthylène-sorbitanne,
du polyoxyéthylène polysorbate monolaurate de sorbitanne, et
de l'alcool polyoxyéthylène oléylique.
